(19) Europäisches Patentamt
European Patent Office
Office européen des brevets

(11) EP 1 656 840 B1

(12) EUROPEAN PATENT SPECIFICATION

(45) Date of publication and mention of the grant of the patent:
**14.05.2008 Bulletin 2008/20**

(51) Int Cl.:
***A23L 3/3436*** *(2006.01)* ***A61K 8/19*** *(2006.01)*
***B01J 13/00*** *(2006.01)* ***C08G 83/00*** *(2006.01)*

(21) Application number: **05024305.4**

(22) Date of filing: **08.11.2005**

(54) **Use of an active oxygen eliminator**

Verwendung eines Mittels zur Beseitigung vom Sauerstoff

Utilisation d'un agent pour l'élimination de l'oxygène

(84) Designated Contracting States:
**DE FR GB**

(30) Priority: **08.11.2004 JP 2004324200**

(43) Date of publication of application:
**17.05.2006 Bulletin 2006/20**

(73) Proprietor: **FUJIFILM Corporation**
**Minato-ku**
**Tokyo (JP)**

(72) Inventor: **Matsunami, Yuki**
**Ashigarakami-gun, Kanagawa (JP)**

(74) Representative: **Rupp, Christian et al**
**Mitscherlich & Partner**
**Patent- und Rechtsanwälte**
**Sonnenstrasse 33**
**80331 München (DE)**

(56) References cited:
**EP-A- 1 598 071**
**WO-A-20/04003558**
**WO-A-20/04037019**
**WO-A-20/05018598**
**WO-A-20/05023467**
**CA-A1- 2 481 982**
**US-A- 5 096 724**
**US-A1- 2002 054 863**

- **PATENT ABSTRACTS OF JAPAN vol. 2000, no. 03, 30 March 2000 (2000-03-30) & JP 11 346715 A (OTSUKA YAKUHIN KOGYO KK), 21 December 1999 (1999-12-21)**
- **PATENT ABSTRACTS OF JAPAN vol. 2000, no. 22, 9 March 2001 (2001-03-09) & JP 2001 122723 A (I BETSUKUSU:KK), 8 May 2001 (2001-05-08)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 656 840 B1

## Description

BACKGROUND OF THE INVENTION

Field of the Invention

The present invention relates to the use of an active oxygen eliminator having high active oxygen elimination effect, as well as foods, drinks, and cosmetics comprising said compound.

Description of the Related Art

Oxygen is an essential substance for life support in living world and plays various roles such as generation of energy in living organisms. It has been known that in the energy generation systems and other reaction systems, oxygen will be active oxygens such as superoxide anion ($\cdot O_2^-$), singlet oxygen ($^1O_2$), and hydroxy anion (.OH) by actions of enzymes, ultraviolet rays, and other radiations.

The active oxygens play an extremely important role for living organisms from the aspect of actions such as bactericidal action by leucocytes, in other words, decomposing foreign bodies preyed on by phagocytes. At the same time, when the active oxygens are exceedingly generated, it damages biomedical tissues and promotes excessive oxidation of unsaturated fatty acids causing formation of phospholipids in biomembranes to trigger generation of inflammatory factors such as lipoperoxides. In addition, active oxygens possibly produce alkoxy radicals and hydroxy radicals in conjunction with the lipoperoxides to attack biomembranes, resulting in membrane damages and deactivation of various useful enzymes.

On the other hand, there are enzymes which deactivate the active oxygens in living organisms. Examples thereof include superoxide dismutases (SOD), catalases, peroxidases, and glutathione peroxidases. Examples of various vitamins having antioxidant effect are α-tocopherols (vitamin E), and the like. Living organisms are normally maintained by actions of these enzymes and vitamins.

However, in the environments surrounding living organisms today, there are a wide variety of chemical substances which promote in vivo production of active oxygens such as herbicides, insecticides, detergents, pharmaceuticals, and various food additives. These chemical substances have effect to generate much more active oxygens, produce and accumulate much more lipoperoxides than the abilities of protecting appropriate mechanisms in living organisms generated by the enzymes and vitamins.

When the active oxygens abnormally increase inside a body, in accordance with chain-like progression of excessive oxidation reactions, it could lead to serious damages such as various inflammations, hepatopathy, renal damages, gastropathy, arteriosclerosis, hemolysis, senile dementia, retinopathy, pulmonary damages, and ischemic vascular diseases.

As mentioned above, it has been recognized that an excessive amount of active oxygens negatively affects living bodies, and it is studied that the active oxygen elimination effect should be positively utilized for daily foods and drinks which are taken into bodies, and for cosmetics which are directly applied to skins. For example, there have been proposed foods and drinks, and cosmetics containing platinum colloids having active oxygen elimination effect (see Japanese Patent Application (JP-A) Nos. 11-346715 and 2001-122723).

Directed to this object of eliminating active oxygen, US 5,096,724 describes a carrier compound immobilized in or on a solid phase. The carrier compound is reactive to bind molecular oxygen and includes a complex formed from a transition metal ion and a multidentate organic chelate. The solid phase is substantially immiscible in or separated from a ligand-containing environment, and the compositions are utilized by contacting the immobilized carrier compounds with the ligand-containing environment. Further, WO 2004/037019 A1 describes specific metal compositions capable of removing active oxygen and comprising one or more metals selected from among gold, platinum, silver and copper.

However, platinum particles relating to these proposals have relatively large particle diameters and are poor in dispersibility, therefore, a satisfying level of active oxygen elimination effect has not yet been obtained, and there have been demands for further improvements and developments in active oxygen eliminators.

Further, US 2002/054863 A1 discloses dendritic polymer conjugates which are useful drug delivery systems for carrying platinum containing anti-tumor agents to malignant tumors. These dendritic polymer conjugates are prepared by obtaining a dendritic polymer having functional groups which are accessible to a platinum containing compound capable of interacting with the functional groups, contacting the dendritic polymer with the platinum containing compound, and reacting the dendritic polymer with the platinum containing compound. More further, WO 2004/003558 A1 describes methods for preparing encapsulated noble metal nanoclusters wherein the nanoclusters are encapsulated by a dendrimer.

## SUMMARY OF THE INVENTION

It is therefore an object of the present invention to provide the use of an active oxygen eliminator which is suitably used for various foods, drinks and various cosmetics.

The invention is further defined by the claims.

An active oxygen eliminator used according to the present invention comprises composite particles in which a platinum group element is bonded to and/or included in dendritic molecules. Since the active oxygen eliminator has smaller particle diameters and a narrower particle size distribution than those of typical platinum nanoparticles and high dispersion stability, it has high active oxygen elimination effect and can be preferably used for foods, drinks, and cosmetics by adding it thereto.

Here, the active oxygen elimination effect includes singlet oxygen elimination effect.

Foods and drinks according to the present invention comprise the active oxygen eliminator used in the present invention.

Here, the foods and drinks widely include general foods, health foods, health-promoting foods, quasi-drugs, and pharmaceuticals all of which are orally taken.

Cosmetics according to the present invention comprise the active oxygen eliminator used in the present invention.

Here, the cosmetics mean various cosmetics which are applied to skins and scalps, for example, cosmetics, medicated cosmetics, and medicinal products.

## BRIEF DESCRIPTION OF THE DRAWINGS

FIG. 1 is a view schematically showing an example of a method of adding a dendritic molecules-containing liquid and a platinum group element-containing liquid at the same time.

FIG. 2 is a view schematically showing another example of a method of adding a dendritic molecules-containing liquid and a platinum group element-containing liquid at the same time.

## DESCRIPTION OF THE PREFERRED EMBODIMENTS

### (Active Oxygen Eliminator and Production Method thereof)

The active oxygen eliminator used in the present invention comprises composite particles in which a platinum group element is bonded to and/or included in dendritic molecules, and further comprises other components in accordance with the necessity.

The production method of the active oxygen eliminator used in the present invention comprises forming composite particles by bonding a platinum group element to dendritic molecules and/or having the platinum group element included in the dendritic molecules, and further comprises other processes in accordance with the necessity.

The active oxygen eliminator used in the present invention is produced in accordance with the production method of the active oxygen eliminator of the present invention.

Hereinafter, the present invention will be described in detail as to the active oxygen eliminator used in the present invention through explanations of the production method of the active oxygen eliminator of the present invention.

### < Formation of Composite Particles >

The forming of the composite particles is a process for forming the composite particles by bonding a platinum group element to dendritic molecules and/ or having the platinum group element included in the dendritic molecules.

Here, examples of aspects of the bond and the inclusion of the platinum group elements include chemical bonds, and bonds by electrostatic interactions, and examples of the chemical bonds include coordinate bonds, ionic bonds, and covalent bonds.

For the forming composite particles, there is an aspect in which the platinum group element is reacted with dendritic molecules having any one of sites capable of complex forming with the platinum group elements and sites to which the platinum group element can be bonded by action of an electrostatic attraction force.

#### - Dendritic Molecules -

The dendritic molecules are highly-branched dendritic molecules having a substantially constant number of

metallic element capture sites and preferably, the dendritic molecules mean monodisperse dendritic molecules.

The dendritic molecules include not only dendrimers and dendrons in which surface groups are regularly and successively branched from the core which is the center of branching but also hyperbranch polymers, and they may also be included into a part of other materials of the active oxygen eliminator. In other words, they may be a substance in which the functional groups on the surface of the dendritic molecules are bonded to polymer molecules or the other materials of the active oxygen eliminator or may be organic molecules containing dendritic molecules. For example, dendritic molecules said in the present invention include molecules of which the surface of dendrimers is bonded to the main chain of a polymer molecule.

The metallic element capture sites (hereinafter, they may be referred to as focal sites) can be suitably selected, for example, they are preferably any one of functional groups capable of bonding to the platinum group elements or functional groups capable of electrostatically interacting with the platinum group elements.

For the bonding types of functional groups capable of bonding to the platinum group elements, there are coordinate bonds, chemical bonds, ionic bonds, covalent bonds, and the like.

Examples of the functional groups capable of coordinately bonding to the platinum group elements include $NH_3$, $RNH_2$, $N_2H_4$, $H_2O$, $OH^-$, $O^{-2}$, ROH, $RO^-$, $R_2O$, $MeCOO^-$, $CO_3^{-2}$, $NO_3^-$, $F^-$, $PhNH_2$, $C_5H_5N$, $N_2$, $NO_2^-$, $SO_3^{-2}$, $Br^-$, $H^-$, $C_2H_4$, $C_4H_6$, $CN^-$, RNC, CO, $SCN^-$, $R_3P$, $(RO)_3P$, $R_3As$, $R_2S$, RSH, $RS^-$, $S_2O_3^{-2}$, and $I^-$. However, in the functional groups stated above, R represents an alkyl group, Ph represents a phenyl group, and Me represents a methyl group.

Examples of the functional groups capable of electrostatically interacting with the platinum group elements include quaternary ammonium salts, and $COO^-$, $PO_4^{3-}$, and $SO_4^{2-}$.

The molecular mass of the dendritic molecules (particularly dendrimers) is preferably 200 or more, and more preferably 1,500 to 100,000. When the molecular mass is less than 200, there may be cases where nanoparticles are not formed inside the dendritic molecules.

The number of generations of the dendritic molecules is preferably the first generation or more, and more preferably the second generation to the tenth generation. When the number of generations is less than the first generation, there may be cases where nanoparticles are not formed inside the dedritic molecules.

Examples of the dendrimers include those described in documents such as "Dendrimers and Dendrons" written by G. R. Newkome, C. N. Morefield, and F. Vögtle, published by WILEY-VCH in 2001; on page 1,010 of "Journal Chemical Society, Chemical Communications" by C. J. Hawker et al (1990); on page 138 of Vol. 29, "Angew. Chem. Int. Ed. Engl." (1990); on page 7,638 of Vol. 112, "Journal American Chemical Society" by C. J. Hawker et al (1990); and on page 1,710 of Vol. 263, "Science" by J. M. J. Frechet (1994).

For the dendrimers, specifically, the following ones represented by (1) to (8) are preferably used.

< Dendrimer (1): Amide Type Dendrimer >

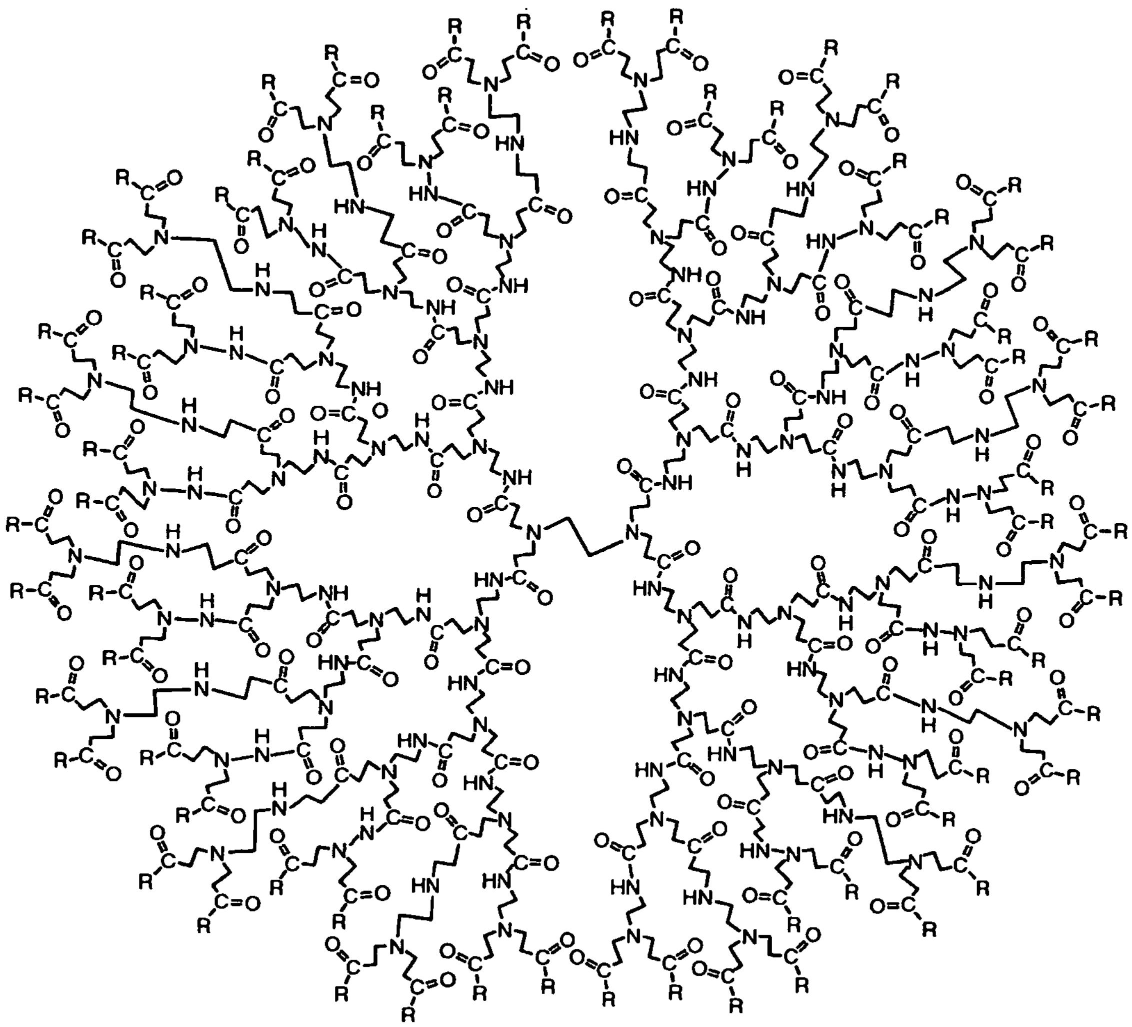

R:

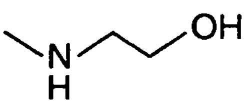

< Dendrimer (2): Amide Type Dendrimer >

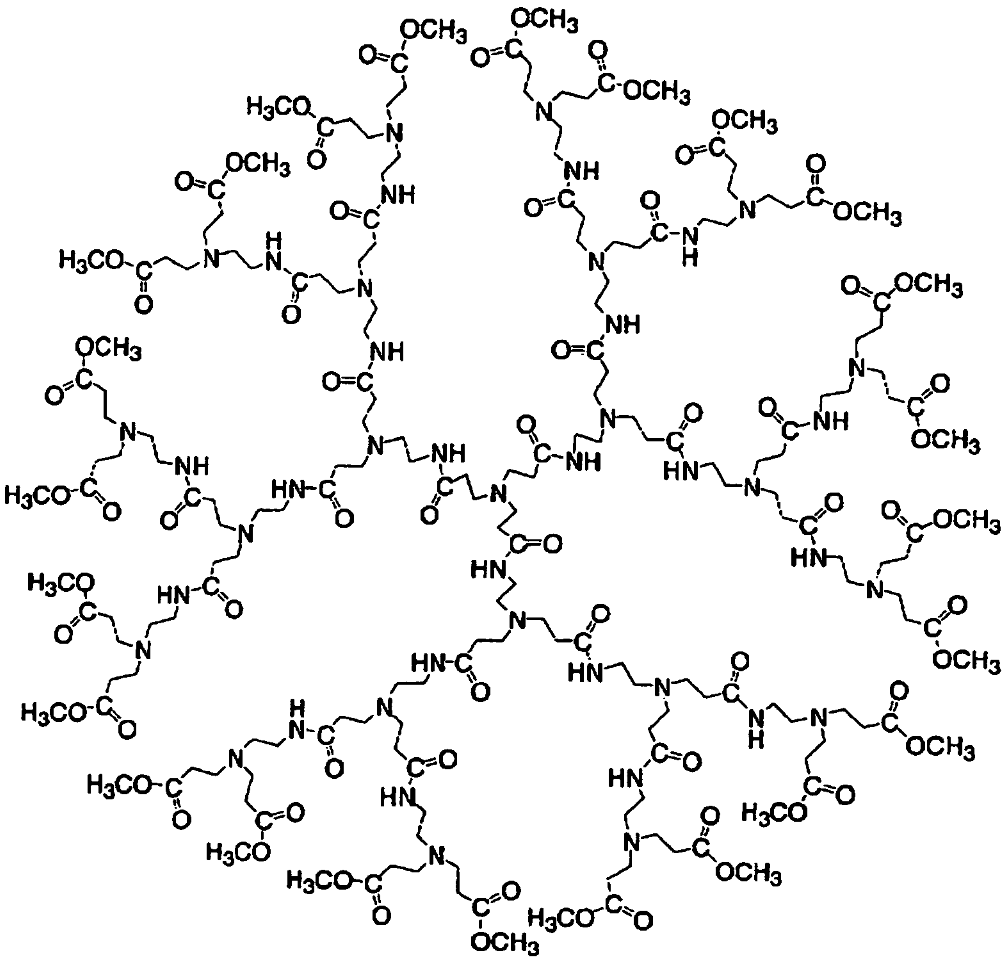

< Dendrimer (3): Amide Type Dendrimer >

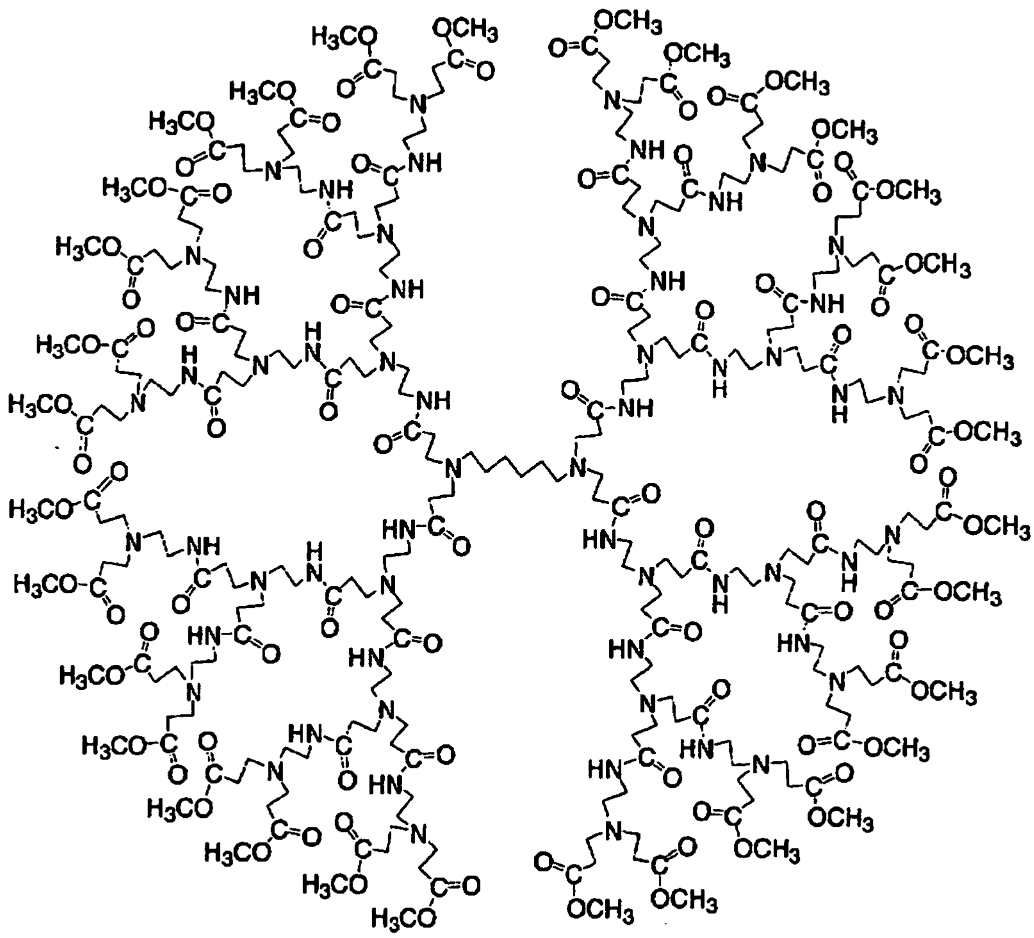

< Dendrimer (4): Amide Type Dendrimer >

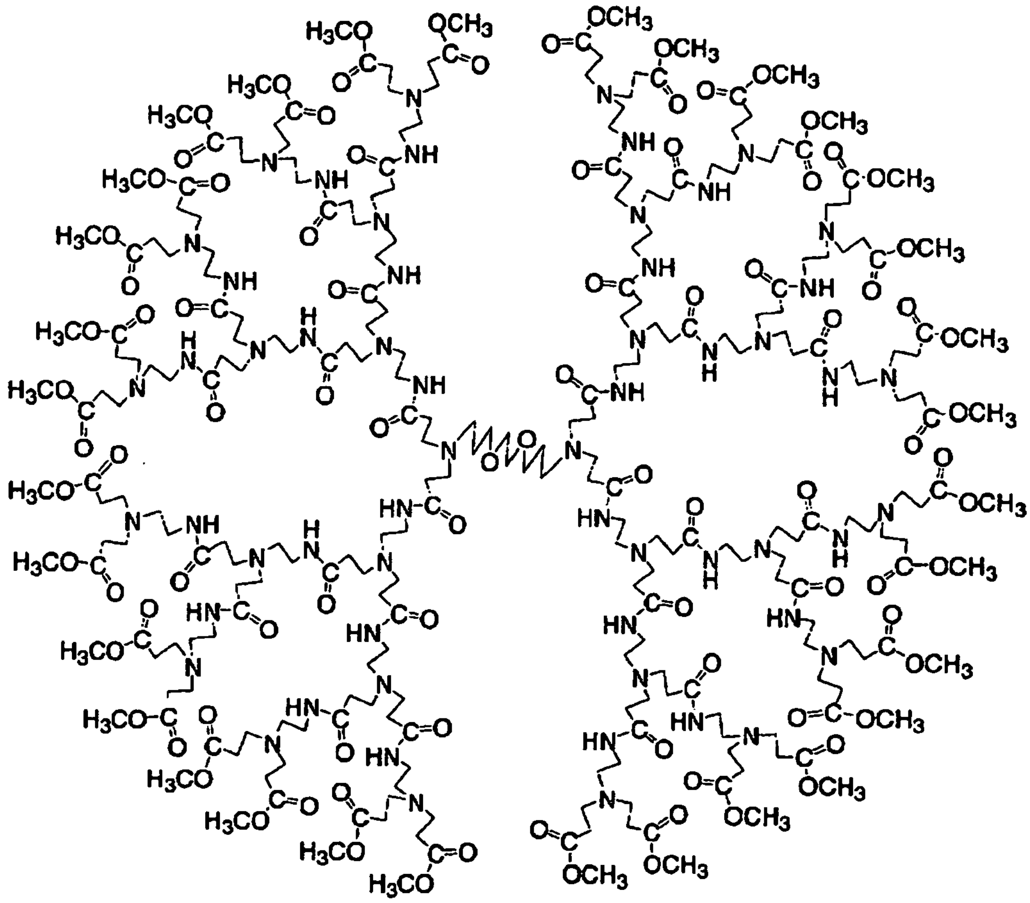

< Dendrimer (5): Propyleneimine Type Dendrimer >

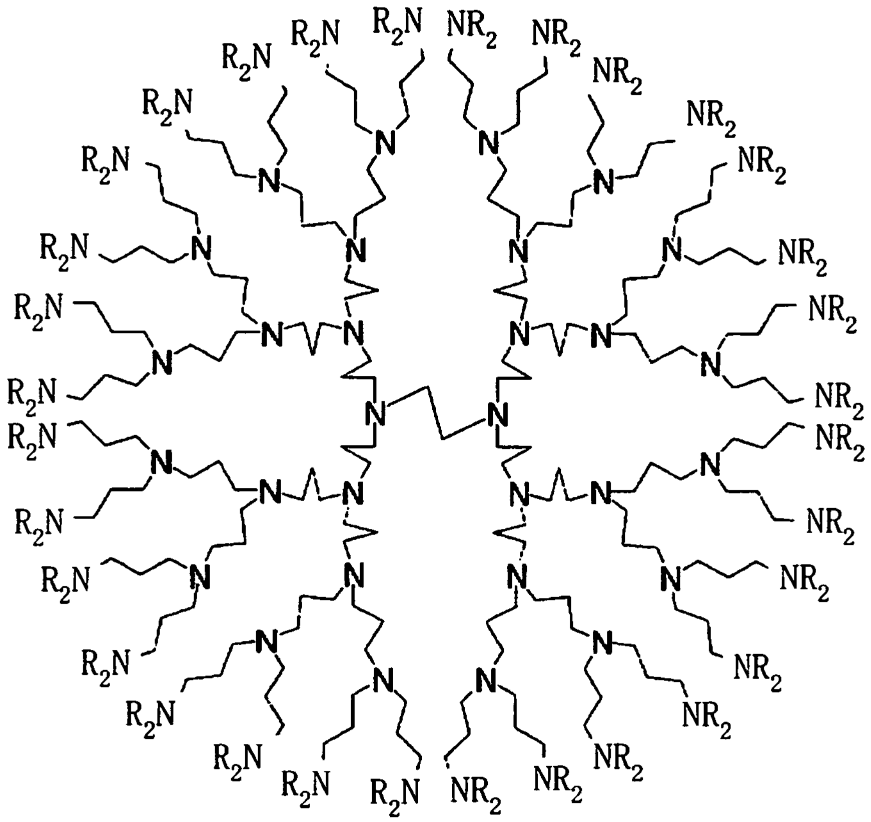

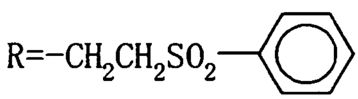

< Dendrimer (6): Propyleneimine Type Dendrimer >

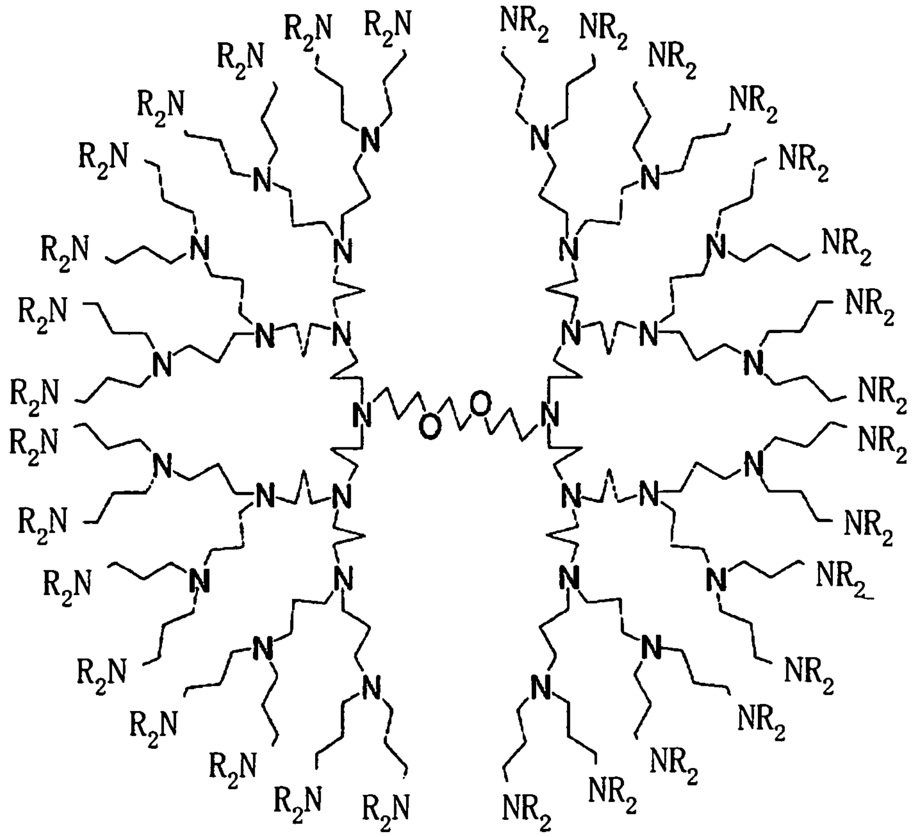

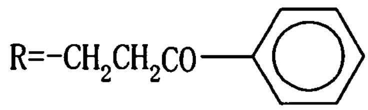

< Dendrimer (7): Propyleneimine Type Dendrimer >

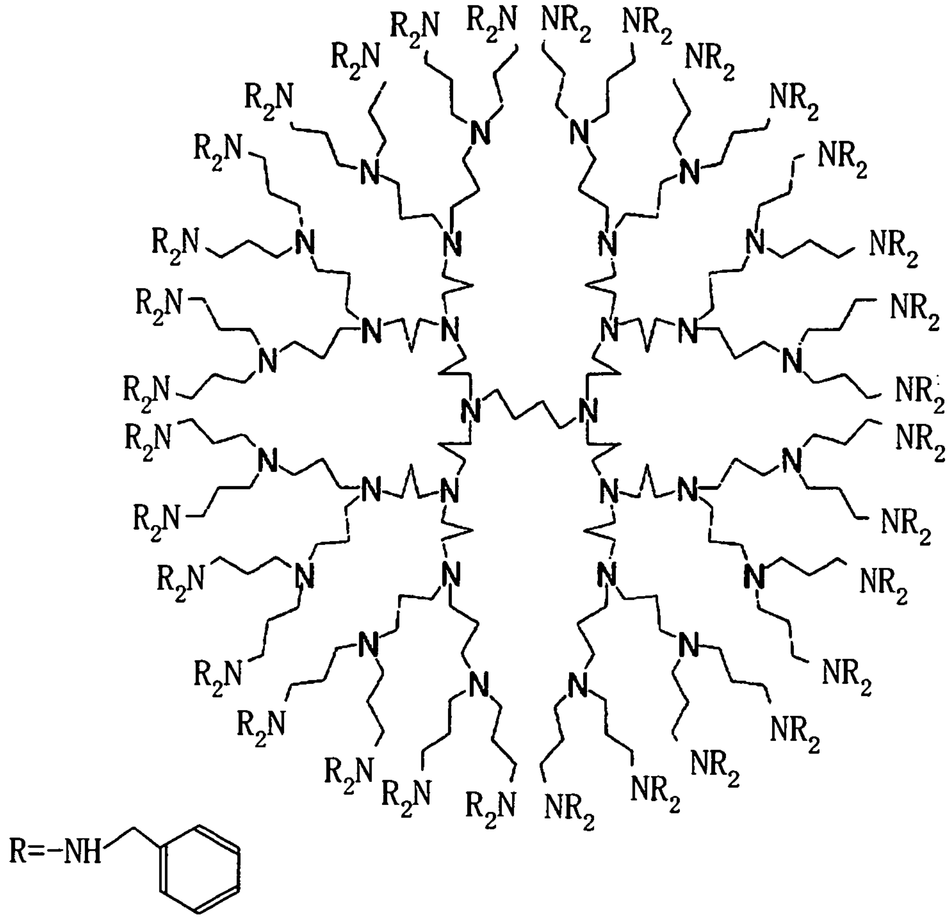

< Dendrimer (8): Methyleneimine Type Dendrimer >

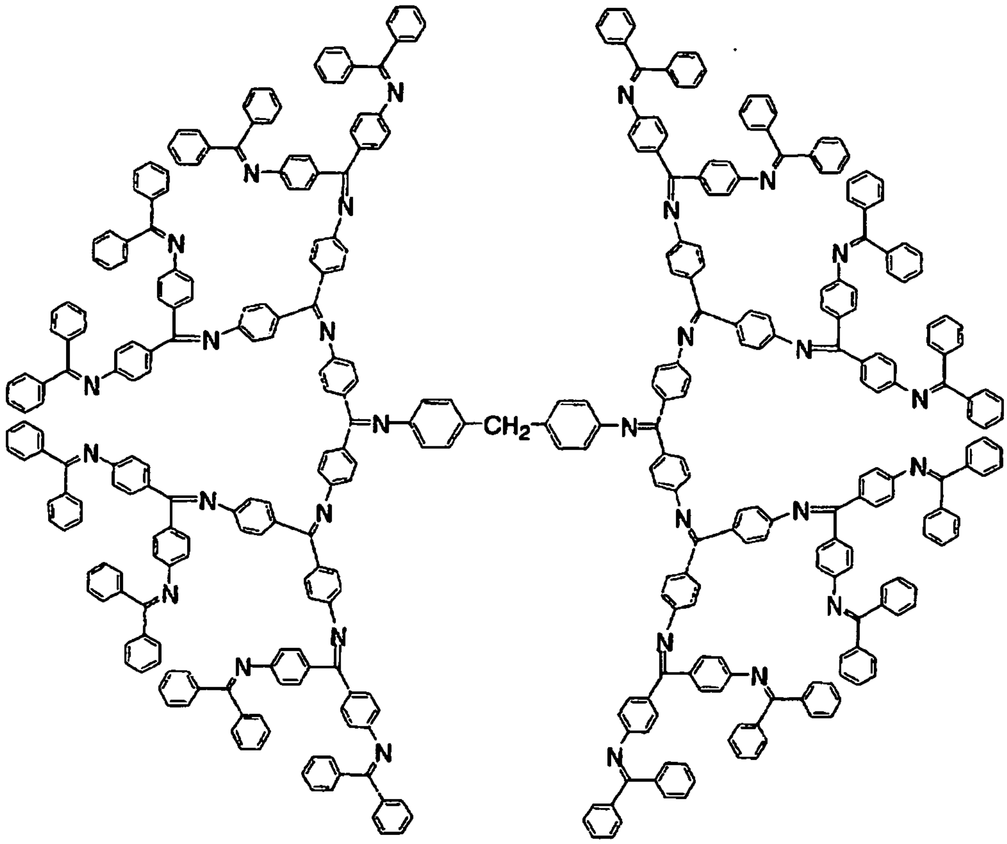

Among the above dendrimers, the production method of a dendrimer containing a trimethyleneimine skeleton is not particularly limited and may be suitably selected in accordance with the intended use. Examples thereof include the following production method.

For example, as disclosed in International Publication Nos. WO/93/14147 and WO/95/02008, there is a method in which a compound containing ammonia and two or more primary amino groups are used as the starting material so as to be reacted with an acrylonitrile group and cyano-ethylated. Thereafter, the acrylonitrile group is reduced to a primary amino group through the use of hydrogen or ammonia in the presence of an active oxygen eliminator (G1). Next, the cyano-ethylation and the reduction to primary amino groups are repeated three times (G2 → G3 → G4) to thereby synthesize a dendrimer containing a trimethyleneimine skeleton. It is noted that G1 represents the first generation of dendrimer, G2 represents the second generation of dendrimer, G3 represents the third generation of dendrimer, and G4 represents the fourth generation of dendrimer, respectively.

In the production method of the dendrimers, it is also possible to use a compound containing one or more functional groups selected from primary amino groups, alcohols, phenols, thiols, tiophenols, and secondary amino groups as the starting material besides ammonia.

Among the above dendrimers, the production method of a dendrimer containing an amidoamine skeleton is not particularly limited and may be suitably selected in accordance with the intended use. Examples thereof include the following production method.

For example, as disclosed in Japanese Patent Publication (JP-B) Nos. 07-2840, 07-57735, and 07-57736, and Japanese Patent Application Laid-Open (JP-A) Nos. 07-267879 and 11-140180, there is a method in which a compound containing a primary amino group is used as the starting material to have the primary amino group reacted with two equivalents of methylacrylate (Michel Addition Reactivity) to thereby yield a bifunctional methyl ester compound having a nitrogen-branched portion. Next, one primary amino group of a diamine compound having a primary amino group is reacted to the methyl ester (exchange reaction between ester and amide), and the other primary amino group is left as

it is (G1). Next, the reaction of which one primary amino group of the diamine compound having the primary amino group is reacted through the reaction with two equivalents of methyl acrylate, and the other primary amino group is left as it is, is repeated three times (G2 → G3 → G4) to thereby synthesize a dendrimer containing an amidoamine skeleton.

In the production method of the dendrimer, it is also possible to use a compound containing one or more functional groups selected from primary amino groups, alcohols, phenols, thiols, tiophenols, and secondary amino groups as the starting material besides ammonia.

Among the dendrimers, the production method of a dendrimer containing a π conjugated polyallyl azomethine skeleton having a branch structure is not particularly limited and may be suitably selected in accordance with the intended use. Examples thereof include the following method.

For example, as described on page 4,414, Vol. 123 of J. Am. Chem. Soc. (2001) written by K. Yamamoto et al. , first, a reactant product (G2) between an amino group of 4, 4' -diamino benzophenon and two equivalents of ketone of the benzophenon is obtained. Next, a reactant product (G3) between the amino group of the 4, 4' -diamino benzophenon and a double amount of the ketone of G2 is obtained. Further, similarly, a reactant product (G4) between the amino group of the 4, 4' -diamino benzophenon and a double amount of the ketone of G3 is obtained, and then a compound containing two amino groups is reacted with a double amount of G4 to thereby synthesize a dendrimer containing a π conjugated polyallyl azomethine skeleton.

The dendrimer containing π conjugated polyallyl azomethine skeleton having a branch structure preferably has a branched structure other than aromatic rings.

The dendrimers may be commercially available products or suitably synthesized ones.

For the dendrons, for example, the following ones represented by (1) to (4) are preferably used. Among them, in order to produce composite particles in a monodisperse condition having a uniform and small particle diameter, dendrons having a mercapto group in the focal site, namely, the dendrons represented by (1) or (3) are preferable. In this case, since the mercapto group in a plurality of focal sites captures one element of the platinum group elements, the obtained composite particles will be formed in reversed micelle and excellent in dispersibility against resins or the like. Further, the composite particles can be easily arrayed due to self-accumulation thereof and will have a sharp particle size distribution.

- Dendron (1) -

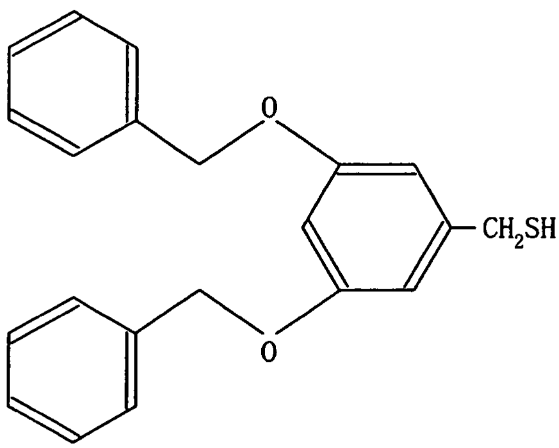

- Dendron (2) -

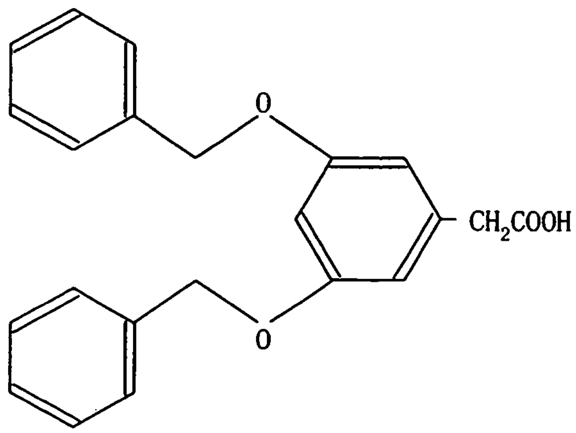

- Dendron (3) -

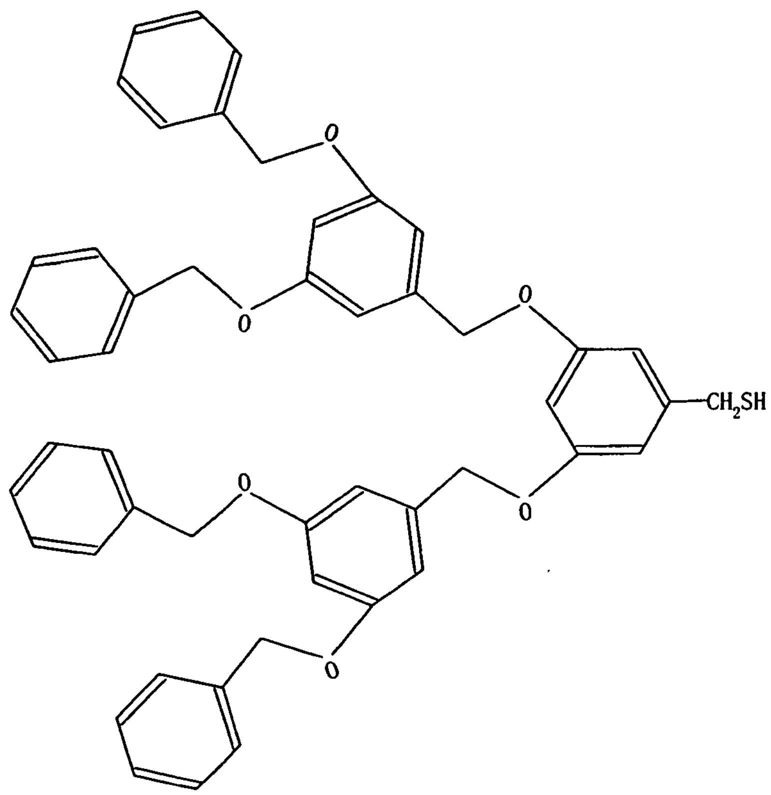

- Dendron (4) -

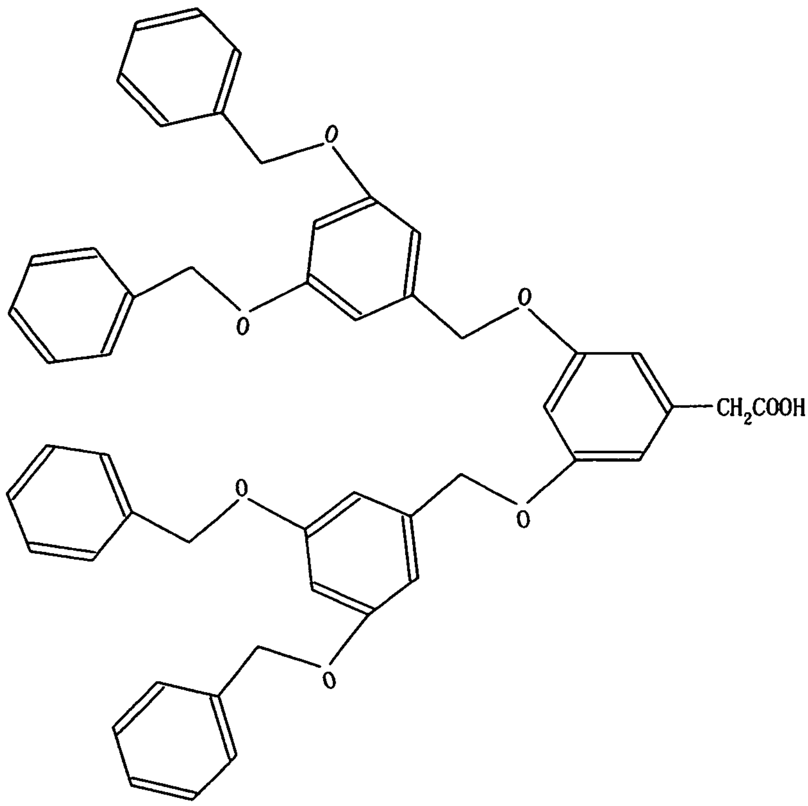

For a method for synthesizing the dendron, for example when the dendron (1) is synthesized, there is a method in which 3, 5-bis[3, 5-bis (benzyloxi) benzyloxi]benzylbromide, tiourea, and a polar solvent are mixed and stirred, and an aqueous solution of sodium hydroxide is further added to the mixture and treated so as to have a pH of 2 to 3 using diluted hydrochloric acid or the like, followed by an extraction of a dendron through the use of ethyl acetate.

The dendron may be commercially available products or suitably synthesized ones.

For the position of focal sites in the dendrons, the focal sites may reside in branched-chains in the dendrons.

For the hyperbranch polymers, for example, the following ones represented by (1) to (2) are preferably used.

< Hyperbranch Polymer (1) >

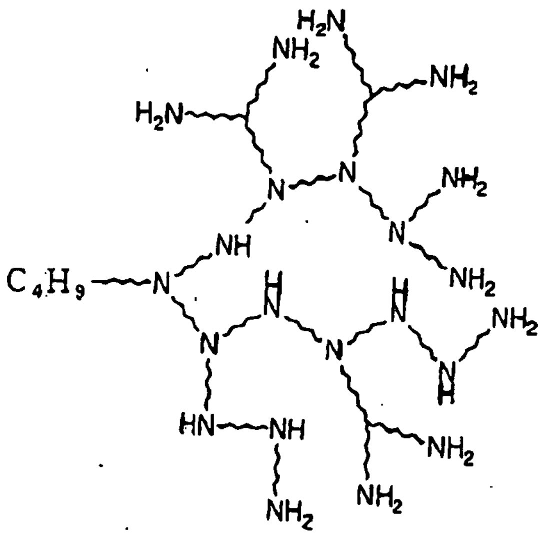

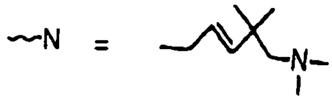

< Hyperbranch Polymer (2) >

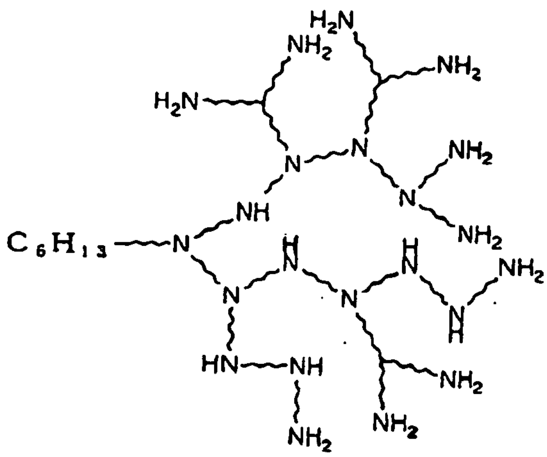

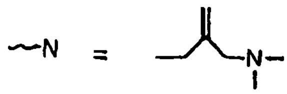

For the production method of the hyperbranch polymers, for example, as described on page 7,071 of Vol. 25 (1992), and page 1,716 of Vol. 31 (1998) of Macromolecules written by M. Suzuki et al., there is a method in which a

primary amine is used as a nucleophilic component to synthesize a hyperbranch polymer by means of ring-opening polymerization of a cyclic compound based on a palladium catalyst.

It is noted that the hyperbranch polymer may be commercially available products or suitably synthesized ones.

Preferably, the dendritic molecules (particularly dendrimers) do not have sites interacting with the platinum group element except for the focal sites. Namely, as shown in the dendrimers (1) to (8), there is no problem with those having focal sites of the platinum group element inside however having no site interacting with the platinum group element on the surface thereof even when left as they are , however, for those having sites interacting with a number of platinum group elements, it is preferred that substituents having a smaller interaction capability (coordination ability) be introduced to the sites interacting with the platinum group elements except for the focal sites to prevent the sites interacting with the platinum group elements except for the focal sites from residing on the surface of the dendritic molecules (dendrimer). In other words, it is preferred that the interaction capability between the focal sites in dendritic molecules and the platinum group elements be greater than the interaction capability between the sites other than the focal sites in the dendritic molecules and the platinum group molecules. It is also preferred that the substituents having a smaller interaction capability have a surface area greater than that of the dendritic molecules. For example, an amino group containing a hydrogen atom is positioned at the terminal of dendritic molecules to be reacted with methyl vinyl ketone, phenyl vinyl ketone, methyl vinyl sulfone, phenyl vinyl sulfone, or the like to thereby introduce substituents having a smaller interaction capability.

Examples of the substituents having a smaller coordination ability include phenyl groups, benzyl groups, and substituted or non-substituted alkyl groups.

It should be noted that when a hard substituent having a benzene ring such as phenyl group or benzyl group is introduced to the surface of the dendrimer, heat resistant ability, stiffness, and light collection ability of the dendrimer and the dendrimer containing particles are respectively enhanced, and such a dendrimer can be suitably used for applications needing these capabilities.

- Platinum Group Elements -

Examples of the platinum group elements include rutheniums (Ru), rhodiums (Rd), palladiums (Pd), osmiums (Os), iridiums (Ir), platinums (Pt), or alloys thereof. Among them, Pt, Pd, PtRu, and PtRd are particularly preferable.

The added amount of the platinum group element is, when the dendritic molecules have focal sites in the molecule chains, preferably equal to or less than the number of focal sites in the dendritic molecules. In doing so, it is possible to effectively form particles without eliminating excessive platinum group elements that are not captured into the dendritic molecules.

When the number of added platinum group elements is more than the number of focal sites in the dendritic molecules, it is preferred that excessive platinum group elements that are not captured into the dendritic molecules be removed and thereafter the platinum group elements that are captured into the dendritic molecules be transformed to particles. In this case, it is preferable in terms of capability of forming seed particles which comprise particles of the focal sites and also in terms of capability of forming particles being substantially uniform in particle size and composition. When the added amount of the platinum group elements is equal to the number of focal sites in the dendritic molecules, it is also preferable in terms of capability of forming particles being substantially uniform in particles size and composition.

Examples of the method of making the platinum group elements captured into all the focal sites in the dendritic molecules include the following methods. When the dendritic molecules have focal sites in branched portions,

(1) a method of which platinum group elements in equal number of the focal sites in the dendritic molecules are added,
(2) a method of which platinum group elements in the number more than the focal sites are added and thereafter the excessive platinum group elements are removed, for example, by means of dialysis.

It may be not only an aspect in which one element of the platinum group elements is captured by one of the focal sites but also an aspect in which one element of the platinum group is captured by two or more of the focal sites.

For the production method of the active oxygen eliminator, when a liquid containing dendritic molecules and a liquid containing platinum group elements are mixed in the formation of the composite particles, it is preferred to drop both liquids at the same time and mix them from the perspective of uniformly forming composite particles having a smaller particle size distribution. In this case, it is preferred that the liquid containing the dendritic molecules in substantially equal quantity of the liquid containing the platinum group elements be mixed. In addition, the liquid containing the dendritic molecules and the liquid containing the platinum group elements be mixed with heating them, and the heating temperature is typically 15°C to 90°C.

A container that the both liquids are poured into and dropped to is not particularly limited, may be suitably selected in accordance with the intended use, and examples thereof include syringes, combinations of syringes and tubes, Y-shaped microtubes, and microreactors.

For the method for sending the both liquids to the container, it may be performed manually, or using a syringe pump, or other units. It is preferred that the both liquids be sent to the container by any one of methods of sending them continuously and sending them intermittently. Sending the both liquids to the container intermittently is particularly preferable because mixing efficiency is improved.

Hereinafter, a specific example of the method for mixing the liquid containing the dendritic molecules and the liquid containing the platinum group elements will be described. For example, as shown in FIG. 1, the liquid containing a dendrimer is poured into syringe A, and the liquid containing the platinum group elements is poured into syringe B, and then the contents of the both syringes are pushed out at the same timing so as to be dropped in a beaker at a constant speed and at the same timing and then stirred. The sending of the both liquids can be performed manually or using a syringe pump, or the like. The dropping rate is typically 0.05mL/min. to 10mL/min. In FIG. 1, a tube may be attached to the ends of the syringes such that the tubes are heated.

As shown FIG. 2, using a tube which is set up in a Y-shaped flow path, the liquid containing the dendrimer is sent from direction of A, the liquid containing the platinum group elements is sent from direction of B to the beaker so as to be dropped at a constant speed and at the same time, and the solution are then stirred. The liquids can be sent manually or using a syringe pump or the like. The sending of the liquids from directions of A and B may be performed continuously or intermittently, however, sending the liquids intermittently from directions A and B enables the liquid containing the dendrimer and the liquid containing platinum group elements to be effectively mixed with uniformity.

It should be noted that the Y-shaped flow path shown in FIG. 2 may be a flow path formed on a substrate made of metals, glasses, silicons, and the like, other than tubes. In the Y-shaped flow path, the part from the joined portion of A direction and B direction to the portion C may be heated. The heating temperature is preferably 15°C to 90°C, and the dropping rate is preferably 0.05mL/ min. to 10mL/min.

Composite particles produced according to the production method of the active oxygen eliminator of the present invention are constant in particle size and are monodisperse particles which are substantially uniform in composition among particles. The particle diameter is not particularly limited, may be suitably selected in accordance with the intended use, and it is preferably 0.1nm to 500nm, more preferably 0.3nm to 100nm, and still more preferably 0.3nm to 10nm. The particles size means the diameter of particles when the particles are formed in a spherical shape, and it means the long side of particles when the particles are formed in a rod shape. The particle size distribution of the composite particles can be narrowed so as to be from zero nanometer to 200nm.

Since the active oxygen eliminator used in the present invention comprises the composite particles and has small particle diameters, a sharp particle distribution and high dispersion stability, it can be used in various areas in various forms such as in powder form, solution form, colloidal solution form, and the like. The active oxygen eliminator has singlet oxygen elimination effect, and in particular, is preferably used for the following foods, drinks, and cosmetics according to the present invention.

(Foods and Drinks)

The foods and drinks of the present invention contain the active oxygen eliminator used in the present invention and further contain other components selected in accordance with the necessity.

The foods and drinks mean those having less potential for harming human health and taken by mouth or from digestive tract in usual social life. The foods and drinks are not limited to classified ones specified by government such as foods, pharmaceuticals, and quasi-drugs, and they mean those widely including, for example, foods, health food products, health-promoting foods, quasi-drugs, and pharmaceuticals all of which are orally taken.

The foods and drinks are not particularly limited and may be suitably selected in accordance with the intended use. Examples thereof include drinks such as soft drinks, carbonated beverages, energy drinks, fruit beverages, and lactic acid drinks; frozen desserts such as ice creams, sherbets, snow cones; soba i.e. noodles such as buckwheat noodles, udon i.e. Japanese wheat noodles, bean-starch vermicelli, jiao-zi wrappers made of wheat flour, wrappers of Chinese-style steamed meat or shrimp dumpling, Chinese noodles, and instant noodles; confectioneries such as hard candies, candies, gums, chocolates, sweet-drops, snack foods, biscuits, jellies, jams, cream foods, baked desserts, and breads; fishery products such as crabs, salmons, clams, tunas, sardines, shrimps, skipjacks, mackerels, whale meat, oysters, sauries, cuttlefish and calamaries, ark shell meat, scallops, abalones, sea urchin roes, salmon roes, and Haliotis; fishery and livestock processed foods such as fish sausages, hams, and sausages; milk products such as processed milk, and fermented milk; fats, fatty oils and oil and fat processed products such as salad oils, vegetable oils, margarines, mayonnaises, shortenings, whip creams, and dressings; flavorings and seasonings such as sources and mop sources; retort pouch foods such as fillings and/or sauces for curry, stew, oyako-don i.e. bowl of rice topped with boiled chicken and eggs, rice gruels, zo-sui i.e. rice and soup boiled with various ingredients such as vegetables, bowl of rice topped with cooked various ingredients with Chinese seasoning, katsu-don i.e. bowl of rice topped with pork cutlet and eggs, ten-don i.e. bowl of rice topped with tempura, una-don i.e. spicy broiled eel bowl, hashed rice, Japanese hotchpotch, Ma-po Dou-fu, gyu-don i.e. bowl of rice topped with seasoned beef, meat source, egg soup, omelet rice, jiao-zi, Chinese-style steamed meat or shrimp dumpling, hamburger, and meatball; health food products and dietary supplements in various forms; pharmaceuticals such as tablets, capsules, stamina drinks, and lozenges; and quasi-drugs.

The other components mentioned above include supplemental raw materials, additives or the like which are typically used in producing the foods and drinks.

The supplemental raw materials and additives are not particularly limited, and may be suitably selected in accordance with the intended use. Examples thereof include glucoses, fructoses, maltoses, sorbitols, steviosides, Rubsoside, corn syrups, lactoses, citric acids, tartaric acids, malic acids, succinic acids, lactic acids, L-ascorbic acids, d1-α-tocopherols, sodium erytohorbates, glycerins, propylene glycols, glycerin fatty acid esters, polyglycerin fatty acid esters, sucrose fatty acid esters, sorbitan fatty acid esters, Arabic gums, carrageenans, caseins, gelatins, pectins, agars, vitamin Bs, nicotinic-acid amides, calcium pantothenates, amino acids, calcium salts, pigments, flavoring ingredients, and preservatives.

The added amount of the active oxygen eliminator used in the the foods and drinks of the present invention varies depending on the type of the foods and drinks to which the active oxygen eliminator is added and cannot be determined unconditionally, however, it may be added in the range as long as original tastes of the foods and drinks are not reduced, and it is preferably 0.001% by mass to 50% by mass, and more preferably 0.01% by mass to 20% by mass. In the case of foods and drinks in the forms of granulation, tablet, or capsule, the added amount is preferably 0.01% by mass to 100% by mass, and more preferably 5% by mass to 100% by mass.

(Cosmetics)

The cosmetics of the present invention contain the active oxygen eliminator used in the present invention and further contain other components which are suitably selected in accordance with the necessity.

The cosmetic may be suitably selected in accordance with the intended use, and examples thereof include ointments, skin creams, milky lotions, lotions, facial masks, jellies, lip creams, lip sticks, hair creams, hair lotions, and bath agents.

The mixed amount of the active oxygen eliminator relative to the total amount of the cosmetic varies depending on the type or the like of the cosmetic, may be accordingly adjusted, and it is preferably 0.001% by mass to 10% by mass, and more preferably 0.01 % by mass to 5% by mass.

In the cosmetics containing the active oxygen eliminator, various main agents, auxiliary agents, and other components which are typically used for producing cosmetics can be further used in accordance with the necessity in the range as long as the effects of the active oxygen eliminator of the present invention are not reduced.

The other components mentioned above are not particularly limited and may be suitably selected in accordance with the intended use. Examples thereof include skin-whitening agents, astringent drugs, pesticides, antibacterial agents, ultraviolet absorbers, moisturizers, cellular stimulants, antiflash agents, antiallergic agents, oils, waxes, hydrocarbons, fatty acids, alcohols, esters, surfactants, and fragrances.

Hereinafter, the present invention will be described in further detail with reference to several examples below.

Example 1

- Preparation of Active Oxygen Eliminator (Composite Particles) -

Using a 50mL conical flask, a 15mmol/L of $K_2$ [$PtCl_4$] aqueous solution in an amount of 10.0mL (15.0 x $10^{-5}$ moles) was transferred on a 20mL dropping funnel. In another 50mL conical flask, 5.0mL of a 0.5mmol/L aqueous solution of dendrimer [product name: Starburst (polyamideamine type dendrimer (PAMAM), manufactured by Aldrich, Inc., Generation 4, having 64 OH groups on the surface thereof] dissolved therein was poured and then stirred with a magnetic stirrer while dropping a platinum ion solution to the dendrimer solution at room temperature for 5 minutes. Thereafter, the solution was stirred for 4 hours at a temperature of 40°C.

With respect to the obtained platinum ion-dendrimer complex and water, the nitrogen atomic mass was measured by an elemental analyzer (2400, manufactured by Parkin Elmer, Inc.), the amount of platinum ion was measured by an atomic absorption spectrometer (Z5010, manufactured by Hitachi Ltd.), and it was verified that the ratio of the amount of the platinum ion and the nitrogen atomic mass was 60:62.

Ultraviolet spectrum of the obtained solution was measured, and a new absorption peak was observed at a wavelength of 260 namometers, the new absorption which had not been observed in the aqueous solution of the polyamideamine type dendrimer and the aqueous solution of $K_2$ [$PtCl_4$].

The reactant solution was washed, and coated on a polyethylene terephthalate (PET) base, and dried to be used as a sample. The sample was then measured by means of ESCA (Electron Spectroscopy for Chemical Analysis), and the measurement showed that the chemical shift value of the Pt-$4f_{7/2}$ in the $K_2$ [$PtCl_4$] aqueous solution was shifted from 73.0eV to 72.5eV, and the chemical shift value of the polyamideamine type dendrimer N-1s was shifted from

398.4eV to 399.3eV.

Example 2

- Preparation of Dendron having a mercapto group in the focal site -

In a vessel, 1.61g (2.0mmol) of 3, 5-Bis[3,5-bis (benxyloxy) benzyloxy] benzyl Bromide, 0.18g (2.4mmol) of tiourea, and 10mL of dimethyl sulfoxide (DMSO) were mixed and then stirred at room temperature overnight to obtain a reactant mixture. To the obtained reactant mixture, 5mL of a 10% by mass sodium hydroxide was added and stirred at room temperature for 1 hour.

Next, the stirred solution was adjusted so as to have a pH 2 to 3 using diluted hydrochloric acid, and then the extraction was performed using ethyl acetate. The obtained ethyl acetate layer was washed with a saturated saline solution and then dried with magnesium sulfate to distill away the solvent and to thereby obtain 1.38g of dendron having a mercapto group in the focal site, represented by the following formula, in the form of oily matter (yield of 92%).

It should be noted that the dendron having a mercapto group in the focal site was identified by measuring $^1$H-NMR spectrum thereof through the use of heavy chloroform as a solvent.

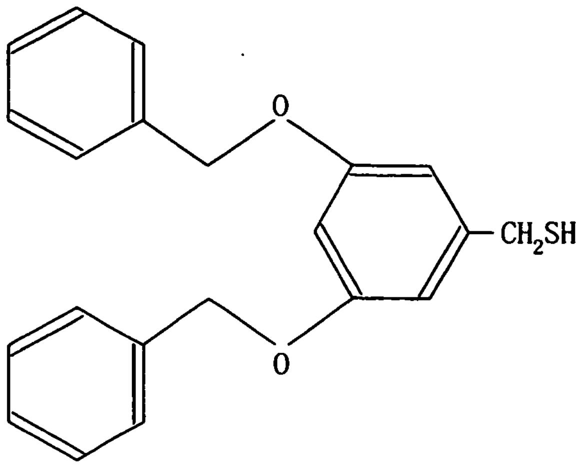

- Preparation of Active Oxygen Eliminator (Composite Particles) -

To a solution in which 2.5mg (7.4mmol) of the dendrons having a mercapto group in the focal site was dissolved in 5mL of ethyl acetate, a solution in which 20.0mg (58.8mmol) of $HPtCl_4$ was dissolved in 5mL of ion-exchange water was added and stirred at room temperature for 5 minutes. To the solution, a solution in which 22.8mg (600mmol) of $NaBH_4$ was dissolved in 5mL of ion-exchange water was added evenly and stirred at room temperature for 5 minutes to thereby obtain platinum composite particles with the dendron coordinated therein.

As a result of observation of the obtained platinum composite particles using a transmission electron microscope (TEM), a space equivalent to two dendron molecules was observed between the platinum nanoparticles. This verified that the platinum nanoparticles are coated with the dendron molecules.

Comparative Example 1

- Preparation of Platinum Nanocolloid Solution (Active Oxygen Eliminator) -

In a 100mL two-aperture eggplant-formed flask to which an Aline condenser and a three-way cock were connected, 0.147g of poly(1-vinyl-2-pyrolidone) (manufactured by Wako Pure Chemical Industries, Ltd.) was poured and dissolved in 23mL of distilled water. The solution was stirred for 10 minutes, and 2mL ($1.66 \times 10^{-2}$ mole/L) of a chloroplatinic acid solution ($H_2PtCl_6 \cdot 6H_2O$, manufactured by Wako Pure Chemical Industries, Ltd.) was added to the solution, and the solution was further stirred for 30 minutes. The reaction system inside the flask was substituted with nitrogen, and 25mL of a highest quality ethanol was added to the reaction liquid, and the reaction liquid was refluxed at 100°C for 2 hours while keeping the nitrogen atmosphere. The UV value of the reaction liquid was measured, and then the completion of the reduction reaction was verified with disappearance of platinum ion peak, and saturation of the peak due to metal

solid-specific scattering.

Next, the organic solvent was removed from the reaction liquid under reduced pressure, and then water was added to the reaction liquid to prepare a platinum nanocolloid solution having a platinum concentration of 1mmole/L). The average particle diameter of the platinum nonocolloid in the obtained platinum nonocolloid solution was 2.4 ± 0.7nm.

Next, with respect to active oxygen eliminators in Examples 1 to 2 and Comparative Example 1 and β-carotene, sodium azide, ascorbic acid, hypotaurine, and mannitol all of which are known as an active oxygen eliminator in the art, the singlet oxygen elimination constant was measured for verifying singlet oxygen elimination effect. Table 1 shows the measurement results. In the measurement of active oxygen eliminators of Examples 1 and 2, a sample having a same platinum concentration as in Comparative Example 1 (1 mmole/L) was used. < Measurement of Singlet Oxygen Elimination Constant >

For a singlet oxygen detector in the measurement, a detector disclosed in Japanese Patent (JP-B) No. 3356517 was used. In the flow cell of the detector, a 200μmole/L of Rose Bengal aqueous solution was circulated at a rate of 20mL/minuted. When a laser at a wavelength of 514.5nm being the absorption wavelength of Rose Bengal was irradiated to the cell, and then a luminescence associated with a transition of singlet oxygen was observed, and the luminescence peak was observed at a wavelength of 1,268nm. Next, the platinum nonocolloid solution was added to the Rose Bengal aqueous solution with various concentrations from 1μmole/L to 10μmole/L, and then the luminescence intensity (I) was measured. The intensity ratio $(I_0)/(I)$ between luminescence intensity (I) and luminescence intensity $(I_0)$ at each of these concentrations was plotted to the concentration (Cq: platinum concentration), and then the singlet oxygen elimination constant (kq) was calculated based on the following Equation 1.

$$I_0/I = 1 + kq \cdot \tau \cdot Cq \qquad \text{Equation 1}$$

In Equation 1 (Stern-Vormer), $I_0$ represents a luminescence intensity which is accompanied by a transition of singlet oxygen in the case where no active oxygen eliminator is contained, I represents a luminescence intensity in the case where an active oxygen eliminator is contained, kq represents a reaction rate constant, τ represents a constant indicating the life time of the singlet oxygen, and Cq represents a density of the active oxygen eliminator.

Table 1

| | Singlet Oxygen Elimination Constant |
|---|---|
| Example 1 | $1.0 \times 10^{10} M^{-1} S^{-1}$ |
| Example 2 | $1.0 \times 10^{10} M^{-1} S^{-1}$ |
| Compara. Example 1 | $1.6 \times 10^{10} M^{-1} S^{-1}$ |
| β-carotene | $7.0 \times 10^{9} M^{-1} S^{-1}$ |
| Sodium azide | $7.9 \times 10^{8} M^{-1} S^{-1}$ |
| Ascorbic acid | $8.3 \times 10^{6} M^{-1} S^{-1}$ |
| Hypotaurine | $1.0 \times 10^{6} M^{-1} S^{-1}$ |
| Mannitol | $1.0 \times 10^{4} M^{-1} S^{-1}$ |

The results shown in Table 1 exemplifies that active oxygen eliminators prepared in Examples 1 and 2 respectively have a higher singlet oxygen elimination constant than β-carotene, sodium azide, ascorbic acid, hypotaurine, and mannitol which are known as active oxygen eliminators in the art, and the active oxygen eliminators prepared in Examples 1 and 2 have excellent singlet oxygen elimination effect at the same level as that of the platinum nonocolloid prepared in Comparative Example 1 and are useful as an active oxygen eliminator. Composition Example 1

- Preparation of Milky Lotion -

A milky lotion having active oxygen elimination effect with the following composition was produced in the conventional manner.

In a vessel, 1.0g of the active oxygen eliminator of Example 1, 4.0g of jojoba oil, 0.1g of placenta extract, 0.1g of ascorbate magnesium phosphate, 2.0g of olive oil, 2.0g of squalane, 2.0g of cetanol, 2.0g of glyceryl monostearate, 2.5g of polyoxyethylene ether (20 E.O.), 2.0g of polyoxyethylene sorbitan oleate (20 E. O.), 3.0g of 1.3-butylene glycol,

0.15g of hinokichiol, 0.15g of fragrance, and purified water were all added so as to be a total amount of 100g.

Composition Example 2

- Preparation of Skin Cream -

A skin cream having active oxygen elimination effect with the following composition was produced in the conventional manner.

In a vessel, 1.0g of the active oxygen eliminator of Example 2, 5.0g of liquid paraffin, 4.0g of white beewax, 3.0g of cetanol, 10.0g of squalane, 2.0g of lanolin, 0.1g of arbutin, 0.1g of lucinol, 0.1g of Koji acid, 0.1g of Conchiolin (Konkiolin), 0.1g of phellodendron bark extract, 1.0g of stearic acid, 1.5g of polyoxyethylene sorbitan oleate (20 E.O.), 3.0g of glyceryl monostearate, 6.0g of 1.3-butylene glycol, 0.05g of methyl parahydroxybenzoate, 0.1g of fragrance, and purified water were all added so as to be a total amount of 100g.

Composition Example 3

- Preparation of Capsule -

Capsules having active oxygen elimination effect with the following composition were produced in the conventional manner.

20.0mg of the active oxygen eliminator of Example 1, 60.0mg of corn starch, 100.0mg of lactose, 10.0mg of calcium lactate, and 10.0mg of hydroxypropyl cellulose (HPC-L) were mixed in the conventional manner and filled in hard gelatin capsules of the size 1.

Composition Example 4

- Preparation of Capsule -

Capsules having active oxygen elimination effect with the following composition were produced in the conventional manner.

20.0mg of the active oxygen eliminator of Example 2, 60.0mg of corn starch, 100.0mg of lactose, 10.0mg of calcium lactate, and 10.0mg of hydroxypropyl cellulose (HPC-L) were mixed in the conventional manner and filled in hard gelatin capsules of the size 1.

The active oxygen eliminator used in the present invention has high active oxygen elimination effect and can be preferably used by adding it to a wide variety of cosmetics such as ointments, skin creams, milky lotions, lotions, facial masks, jellies, lip creams, lip sticks, hair creams, hair lotions, and bath agents as well as to a wide variety of foods and drinks which are orally taken such as general foods, health foods, health-promoting foods, quasi-drugs, and pharmaceuticals.

## Claims

1. Use of composite particles which comprise a platinum group element and dendritic molecules, wherein the platinum group element is bonded to the dentritic molecules and/or included in the dentritic molecules, said composite particles being used for the elimination of active oxygen in non-therapeutic applications.

2. Use of composite particles for the manufacture of an active oxygen eliminator,
wherein said composite particles comprise a platinum group element and dendritic molecules, the platinum group element being bonded to the dentritic molecules and/or included in the dentritic molecules.

3. Use of composite particles according to claim 1 or 2,
wherein the platinum group element is at least one selected from Pt, Pd, PtRu, and PtRd.

4. Use of composite particles according to any one of claims 1 to 3,
wherein the composite particles have sites of the dendritic molecules to which the platinum group element is bonded and/or in which the platinum group element is included, and the sites are metallic element capture sites.

5. Use of composite particles according to any one of claims 1 to 4,

wherein the dendritic molecules are at least one selected from dendrimers, dendrons, and hyperbranch polymers.

6. Use of composite particles according to any one of claims 1 to 5, wherein the dendritic molecules are contained in a part of the other materials of the active oxygen eliminator.

7. Use of composite particles according to any one of claims 1 to 6, wherein the molecular mass of the dendritic molecules is 200 or more.

8. Use of composite particles according to any one of claims 1 to 7, wherein the number of generations of the dendritic molecules is the first generation or more.

9. Use of composite particles according to any one of claims 1 to 8, wherein the volume average particle diameter D50 of the composite particles is 0.1nm to 500nm.

10. Use of composite particles according to any one of claims 1 to 9 having singlet oxygen elimination effect.

11. Foods and drinks comprising:

an active oxygen eliminator comprising composite particles which comprise a platinum group element and dendritic molecules, wherein the platinum group element is bonded to the dendritic molecules and/ or included in the dendritic molecules.

12. Cosmetics comprising:

an active oxygen eliminator comprising composite particles which comprise a platinum group element and dendritic molecules,wherein the platinum group element is bonded to the dendritic molecules and/ or included in the dendritic molecules.

## Patentansprüche

1. Verwendung von Kompositpartikeln, welche ein Element der Platingruppe und dendritische Moleküle umfassen, wobei das Element der Platingruppe an die dendritischen Moleküle gebunden ist und/oder in den dendritischen Molekülen enthalten ist, wobei die Kompositpartikel zur Eliminierung von Aktivsauerstoff in nicht therapeutischen Anwendungen verwendet werden.

2. Verwendung von Kompositpartikeln für die Herstellung eines Aktivsauerstoffeliminators, wobei die Kompositpartikel ein Element der Platingruppe und dendritische Moleküle umfassen, wobei das Element der Platingruppe an die dendritischen Moleküle gebunden ist und/oder in den dendritischen Molekülen enthalten ist.

3. Verwendung von Kompositpartikeln gemäß Anspruch 1 oder 2, wobei das Element der Platingruppe zumindest eines ist, welches ausgewählt ist aus Pt, Pd, PtRu und PtRd.

4. Verwendung von Kompositpartikeln gemäß irgendeinem der Ansprüche 1 bis 3, wobei die Kompositpartikel Plätze der dendritischen Moleküle aufweisen, an welchen das Element der Platingruppe gebunden ist und/oder in welchen das Element der Platingruppe enthalten ist, und wobei die Plätze Metallelementaufnahmeplätze sind.

5. Verwendung von Kompositpartikeln gemäß irgendeinem der Ansprüche 1 bis 4, wobei die dendritschischen Moleküle zumindest eines aus einem von Dendrimeren, Dendronen und Hyperbranch-Polymeren ausgewählt sind.

6. Verwendung von Kompositpartikeln gemäß irgendeinem der Ansprüche 1 bis 5, wobei die dendritischen Moleküle in einem Teil der anderen Materialien des Aktivsauerstoffeliminators enthalten sind.

7. Verwendung von Kompositpartikeln gemäß irgendeinem der Ansprüche 1 bis 6, wobei die Molekülmasse der dendritischen Moleküle 200 oder mehr beträgt.

8. Verwendung von Kompositpartikeln gemäß irgendeinem der Ansprüche 1 bis 7, wobei die Anzahl von Generationen der dendritischen Moleküle die erste Generation oder mehr ist.

9. Verwendung von Kompositpartikeln gemäß irgendeinem der Ansprüche 1 bis 8, wobei der Durchschnittsvolumenpartikeldurchmesser D50 der Kompositpartikel 0.1 nm bis 500 nm beträgt.

10. Verwendung von Kompositpartikeln gemäß irgendeinem der Ansprüche 1 bis 9, welche einen Singulettsauerstoffeliminierungseffekt aufweisen.

11. Speisen und Getränke, umfassend:

einen Aktivsauerstoffeliminator, welcher Kompositpartikel umfasst, welche ein Element der Platingruppe und dendritische Moleküle umfassen, wobei das Element der Platingruppe an die dendritischen Moleküle gebunden ist und/oder in den dendritischen Molekülen enthalten ist.

12. Kosmetik, umfassend:

einen Aktivsauerstoffeliminator, welcher Kompositpartikel umfasst, welche ein Element der Platingruppe und dendritische Moleküle umfassen, wobei das Element der Platingruppe an die dendritischen Moleküle gebunden ist und/oder in den dendritischen Molekülen enthalten ist.

## Revendications

1. Utilisation de particules composites qui comprennent un élément du groupe du platine et des molécules dendritiques, où l'élément du groupe du platine est lié aux molécules dendritiques et/ou inclus dans les molécules dendritiques, lesdites particules composites étant utilisées pour l'élimination d'oxygène actif dans des applications non thérapeutiques.

2. Utilisation de particules composites pour la fabrication d'un agent éliminant l'oxygène actif où lesdites particules composites comprennent un élément du groupe du platine et des molécules dendritiques, l'élément du groupe du platine étant lié aux molécules dendritiques et/ou inclus dans les molécules dendritiques.

3. Utilisation de particules composites selon la revendication 1 ou 2 où l'élément du groupe du platine est au moins un choisi parmi Pt, Pd, PtRu et PtRd.

4. Utilisation de particules composites selon l'une quelconque des revendications 1 à 3 où les particules composites ont des sites des molécules dendritiques auxquels l'élément du groupe du platine est lié et/ou dans lesquels l'élément du groupe du platine est inclus, et les sites sont des sites de capture d'éléments métalliques.

5. Utilisation de particules composites selon l'une quelconque des revendications 1 à 4 où les molécules dendritiques sont au moins l'une choisie parmi les dendrimères, les dendrons et les polymères hyper-ramifiés.

6. Utilisation de particules composites selon l'une quelconque des revendications 1 à 5 où les molécules dendritiques sont contenues dans une partie des autres substances de l'agent d'élimination de l'oxygène actif.

7. Utilisation de particules composites selon l'une quelconque des revendications 1 à 6 où la masse moléculaire des molécules dendritiques est 200 ou plus.

8. Utilisation de particules composites selon l'une quelconque des revendications 1 à 7 où le nombre de générations des molécules dendritiques est la première génération ou plus.

9. Utilisation de particules composites selon l'une quelconque des revendications 1 à 8 où le diamètre particulaire moyen en volume D50 des particules composites est 0,1 nm à 500 nm.

10. Utilisation de particules composites selon l'une quelconque des revendications 1 à 9 ayant un effet d'élimination de l'oxygène singulet.

11. Aliments et boissons comprenant :

un agent éliminant l'oxygène actif comprenant des particules composites qui comprennent un élément du groupe

du platine et des molécules dendritiques, où l'élément du groupe du platine est lié aux molécules dendritiques et/ou est inclus dans les molécules dendritiques.

**12.** Cosmétiques comprenant :

Un agent éliminant l'oxygène actif comprenant des particules composites qui comprennent un élément du groupe du platine et des molécules dendritiques, où l'élément du groupe du platine est lié aux molécules dendritiques et/ou est inclus dans les molécules dendritiques.

FIG. 1

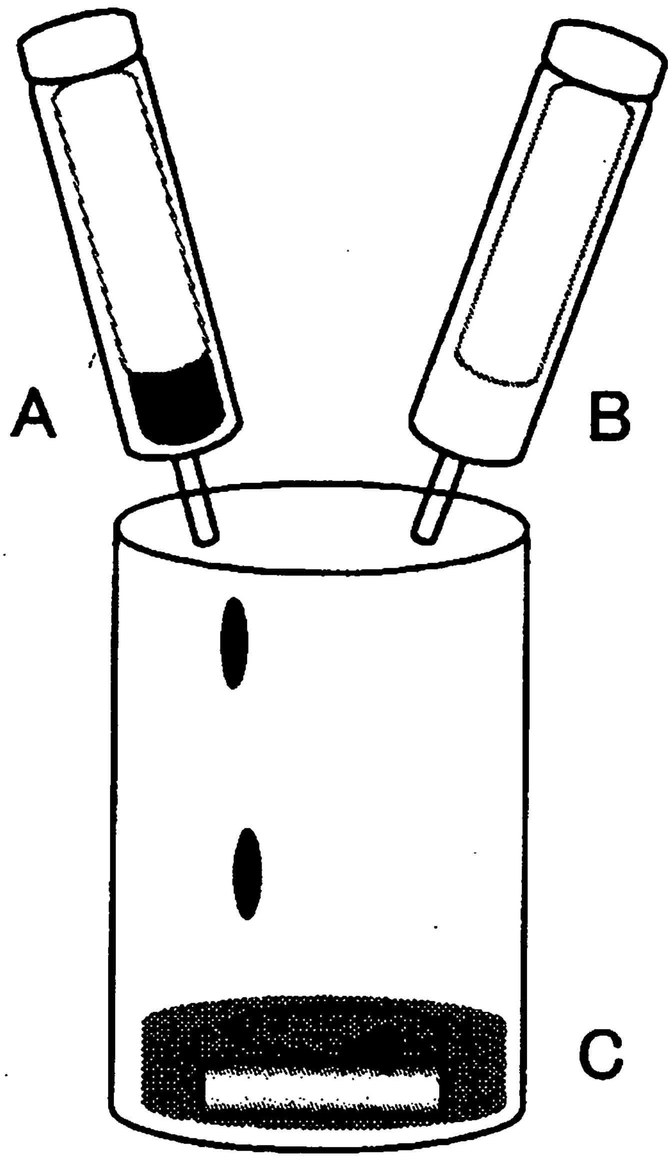

FIG. 2

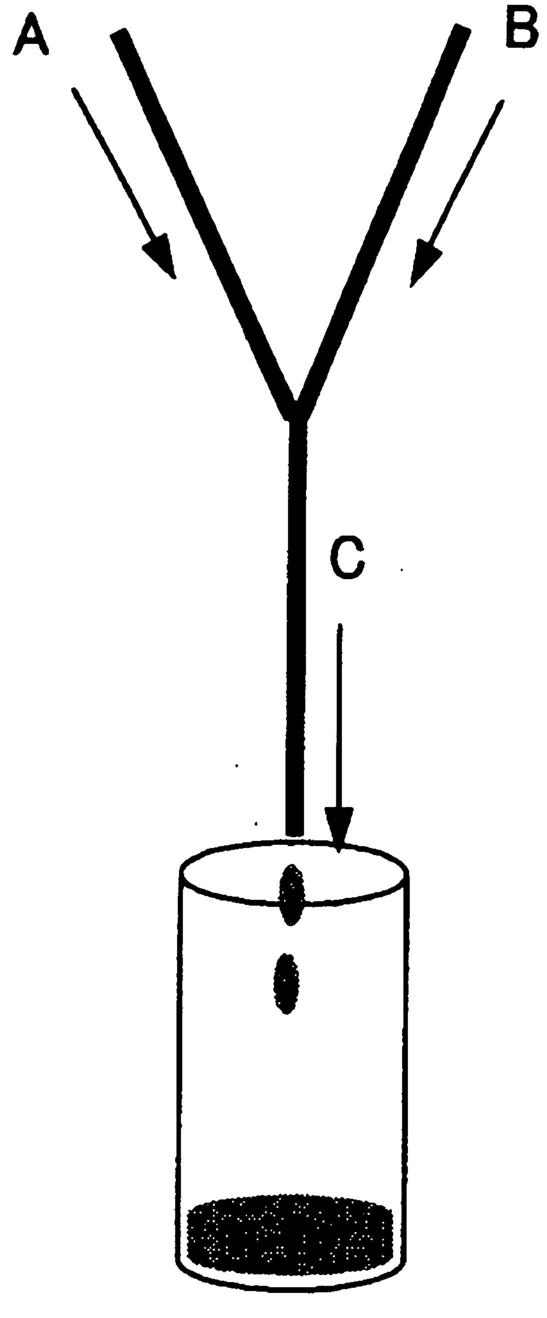

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11346715 A
- JP 2001122723 A
- US 5096724 A
- WO 2004037019 A1
- US 2002054863 A1
- WO 2004003558 A1
- WO 9314147 A
- WO 9502008 A
- JP 7002840 B
- JP 7057735 B
- JP 7057736 B
- JP 7267879 A
- JP 11140180 A
- JP 3356517 B


### Non-patent literature cited in the description


- **G. R. NEWKOME ; C. N. MOREFIELD ; F. VÖGTLE.** Dendrimers and Dendrons. WILEY-VCH, 2001
- **C. J. HAWKER et al.** *Journal Chemical Society, Chemical Communications,* 1990, 1, 010
- *Angew. Chem. Int. Ed. Engl.,* 1990, vol. 29, 138
- **C. J. HAWKER et al.** *Journal American Chemical Society,* 1990, vol. 112, 7, 638
- **J. M. J. FRECHET.** *Science,* 1994, vol. 263, 1, 710
- **K. YAMAMOTO.** *J. Am. Chem. Soc.,* 2001, vol. 123, 4, 414
- *MACROMOLECULES,* 1992, vol. 25, 7, 071
- **M. SUZUKI.** *Macromolecules,* 1998, vol. 31, 1, 716